# FASCICULE DE BREVET EUROPEEN

(11) **EP 1 325 731 B1**
(45) Date de publication et mention de la délivrance du brevet: **21.03.2007**
(21) Numéro de dépôt: 02293269.3
(22) Date de dépôt: 30.12.2002
(51) Int. Cl.: A01N 37/40, A61K 8/34, A61K 8/368, A61K 8/41, A61Q 5/02

(54) **Agent microbicide et composition de traitement cosmétique le contenant**
Mikrobizides Mittel und dieses enthaltende Zusammensetzung zur kosmetischen Behandlung
Microbicidal agent and cosmetic treatment composition containing the same

(30) Priorité: 07.01.2002 FR 0200125
(43) Date de publication de la demande: 09.07.2003
(73) Titulaire: L'ORÉAL, 75008 Paris (FR)
(72) Inventeur: Müller, Rainer, 76344 Leopoldshafen (DE)
(74) Mandataire: Casalonga, Axel

(56) Documents cités:
- EP-A- 1 080 715
- WO-A-87/06827
- FR-A- 2 271 808
- A. HUNTING: "Encyclopedia of shampoo ingredients" 1985 , MICELLE PRESS , CRANFORD, US XP002234592 145850 * page 51, colonne 1, alinéa 2 *
- DATABASE CHEMICAL ABSTRACTS [en ligne] retrieved from STN Database accession no. 133:101859 XP002234593 & T. ISHIMARU ET AL.: "Effect of sugar alcohol on the bactericidal activity of benzalkonium chloride with surfactant" BOKIN BOBAI, vol. 28, no. 2, 2000, pages 81-86, JP

## Description

La présente invention est relative à un agent microbicide et à une composition de traitement des matières kératiniques le contenant.

Dans les compositions cosmétiques se développent au cours du temps des contaminants tels que des bactéries et des champignons. Ces contaminants peuvent être sources d'allergies.

Pour éviter ce type de problème lié à ces contaminants, il est bien connu d'utiliser des conservateurs tels que le propylparaben, l'acide benzoïque, les crésols ou le formaldéhyde, mais ils peuvent eux-mêmes provoquer des allergies.

L'Encyclopedia of shampoo ingrédients (A. Hunting), 1985, page 51, décrit une composition de shampoing comprenant du butylèneglycol et des agents conservateurs tels que le méthyl- et le propyl-paraben, et l'imidazolidinylurée.

De FR 2 271 808, il est également connu des articles de toilette en non-tissé, imprégnés d'un agent de nettoyage moussant et éventuellement d'un agent conservateur.

Il existe donc un besoin pour des produits cosmétiques tels que les shampoings, qui ne contiennent pas ou peu de conservateurs afin de limiter de manière importante les risques d'allergies.

Dans sa recherche portant sur un nouvel agent microbicide, la Demanderesse a découvert de manière surprenante qu'en associant l'acide anisique avec un polyol et un tensioactif cationique, on évitait une contamination microbiologique importante.

La présente invention a donc pour objet un agent microbicide comprenant l'acide anisique, un de ses sels ou un de ses esters d'alkyle en C₁-C₄, au moins un polyol et au moins un tensioactif cationique.

L'invention a encore pour objet une composition de traitement cosmétique le contenant.

D'autres objets, caractéristiques, aspects et avantages de l'invention apparaîtront encore plus clairement à la lecture de la description et des divers exemples qui suivent.

Selon l'invention, l'agent microbicide comprend l'acide anisique, un de ses sels ou un de ses esters d'alkyle en C₁-C₄, au moins un polyol présentant une chaîne hydrocarbonée qui comporte au moins 4 atomes de carbone et qui porte au moins deux groupes hydroxy, et au moins un tensioactif cationique.

L'acide anisique utilisé dans la présente invention peut être utilisé tel quel ou se trouver sous la forme d'un sel de métal alcalin ou alcalino-terreux, d'un sel d'ammonium ou d'un sel avec une amine organique, ou encore sous la forme d'un ester d'alkyle en C₁₋₄. Le groupe alkyle de l'ester peut être linéaire ou ramifié et on peut mentionner à titre d'exemples, les groupes méthyle, éthyle, n-propyle, isopropyle, n-butyle et tertio-butyle.

A titre d'exemples de sel ou d'ester d'acide anisique, on peut notamment citer l'anisate de sodium, l'anisate de potassium, l'anisate de méthyle, l'anisate d'éthyle, l'anisate de propyle ou l'anisate de butyle.

De préférence, l'agent microbicide contient d l'acide anisique.

L'acide anisique, le sel ou l'ester de cet acide est notamment contenu en une quantité de 1 à 30 % en poids, de préférence de 4 à 20 % en poids par rapport au poids total de l'agent microbicide, ou encore en une quantité allant jusqu'à 1 % en poids, de préférence en une quantité de 0,1 à 0,4 % en poids par rapport au poids total d'une composition contenant l'agent microbicide.

Par polyol, on entend un alcool qui présente une chaîne hydrocarbonée comportant au moins 4 atomes de carbone, de préférence de 4 à 50 atomes de carbone, et portant au moins deux groupes hydroxy, de préférence de 2 à 10 groupes hydroxy. Les polyols généralement utilisés dans la présente invention présentent une masse moléculaire moyenne en poids inférieure ou égale à 1000, de préférence comprise entre 90 et 500.

Comme polyol utilisable selon la présente invention, on peut notamment citer un polyalkylèneglycol, tel que les polyéthylèneglycols, notamment le PEG-4, le PEG-6 et le PEG-8, les pentanediols et en particulier le 1,2-pentanediol, le sorbitol ou leurs mélanges ; le pentanediol-1,2, seul ou en mélange avec d'autres polyols, étant particulièrement préféré.

L'agent microbicide selon l'invention comprend de préférence jusqu'à 50 % en poids, mieux encore de 10 à 30 % en poids d'au moins un polyol tel que mentionné ci-dessus, par rapport au poids total d'agent microbicide. Le(s) polyol(s) représente(nt) jusqu'à 10 % en poids, mieux encore 5 à 10 % en poids du poids total d'une composition contenant l'agent microbicide.

L'agent microbicide selon l'invention comprend également un ou plusieurs tensioactifs cationiques bien connus en soi, tels que les sels d'amines grasses primaires, secondaires ou tertiaires, éventuellement polyoxyalkylénées, les sels d'ammonium quaternaire, ou leurs mélanges.

Les amines grasses sont notamment la stéaramidopropyldiméthylamine ou la béhénylamidopropyldiméthylamine.

A titre de sels d'ammonium quaternaire, on peut notamment citer, par exemple :
- ceux qui présentent la formule générale (I) suivante : 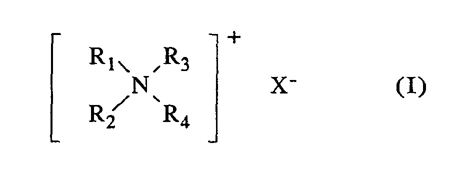 dans laquelle les symboles R₁ à R₄, qui peuvent être identiques ou différents, représentent un groupe aliphatique, linéaire ou ramifié, comportant de 1 à 30 atomes de carbone, ou un groupe aromatique tel que aryle ou alkylaryle. Les groupes aliphatiques peuvent comporter des hétéroatomes tels que notamment les atomes d'oxygène, d'azote, de soufre, de phosphore et d'halogène. Les groupes aliphatiques sont, par exemple, choisis parmi les groupes alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans l'ensemble constitué par les halogénures, les phosphates, les acétates, les lactates, les alkyl(C₂-C₆)sulfates, les alkyl- ou alkylaryl-sulfonates ;
- les sels d'ammonium quaternaire de l'imidazoline comme, par exemple, ceux de formule (II) suivante : 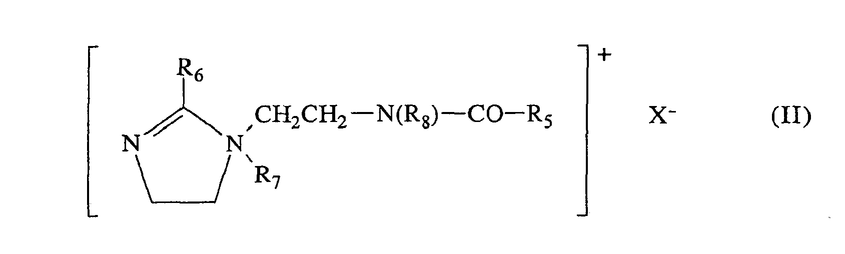 dans laquelle R₅ représente un groupe alcényle ou alkyle comportant de 8 à 30 atomes de carbone, par exemple dérivé des acides gras du suif, R₆ représente un atome d'hydrogène, un groupe alkyle en C₁-C₄ ou un groupe alcényle ou alkyle comportant de 8 à 30 atomes de carbone, R₇ représente un groupe alkyle en C₁-C₄, R₈ représente un atome d'hydrogène, un groupe alkyle en C₁-C₄, X⁻ est un anion choisi dans l'ensemble constitué par les halogénures, les phosphates, les acétates, les lactates, les alkylsulfates, les alkyl- ou alkylaryl-sulfonates. De préférence, R₅ et R₆ désignent un mélange de groupes alcényle ou alkyle comportant de 12 à 21 atomes de carbone, par exemple dérivés des acides gras du suif, R₇ désigne un groupe méthyle, R₈ désigne un atome d'hydrogène. Un tel produit est, par exemple, celui connu sous la dénomination INCI Quarternium-27 (Dictionnaire CTFA, 8^{ème} édition, 2000, volume 2) et commercialisé sous la dénomination REWOQUAT^{®} W 75 par la société REWO.
- les sels de diammonium quaternaire de formule (III) : 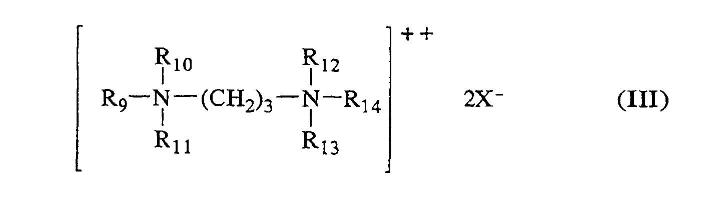 dans laquelle R₉ désigne un groupe aliphatique comportant environ de 16 à 30 atomes de carbone, R₁₀, R₁₁, R₁₂, R₁₃ et R₁₄, identiques ou différents, représentent chacun un atome d'hydrogène ou un groupe alkyle comportant de 1 à 4 atomes de carbone, et X⁻ est un anion choisi dans l'ensemble constitué par les halogénures, les acétates, les phosphates, les nitrates et les méthylsulfates. De tels sels de diammonium quaternaire comprennent notamment le dichlorure de propanesuif-diammonium.
- les sels d'ammonium quaternaire contenant au moins une fonction ester, tels que ceux de formule (IV) suivante : 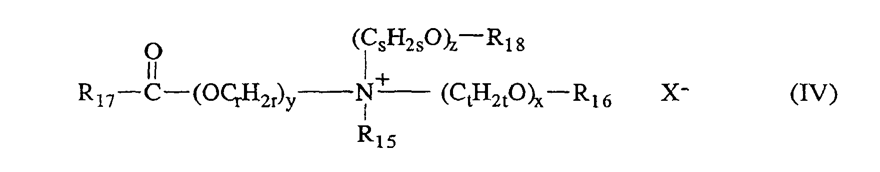
dans laquelle :
R₁₅ est choisi parmi les groupes alkyles en C₁-C₆ et les groupes hydroxyalkyles ou dihydroxyalkyles en C₁-C₆ ;
R₁₆ est choisi parmi :
   - le radical 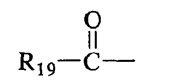
   - les groupes R₂₀ hydrocarbonés en C₁-C₂₂, linéaires ou ramifiés, saturés ou insaturés,
   - l'atome d'hydrogène,
R₁₇ est choisi parmi :
   - le radical 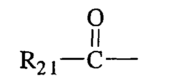
   - les groupes R₂₂ hydrocarbonés en C₁-C₆, linéaires ou ramifiés, saturés ou insaturés,
   - l'atome d'hydrogène,
R₁₇, R₁₉ et R₂₁, identiques ou différents, sont choisis parmi les groupes hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

Les groupes alkyles R₁₅ peuvent être linéaires ou ramifiés, et plus particulièrement linéaires.

De préférence, R₁₅ désigne un groupe méthyle, éthyle, hydroxyéthyle ou dihydroxypropyle, et plus particulièrement un groupe méthyle ou éthyle.

Avantageusement, la somme x + y + z vaut de 1 à 10.

Lorsque R₁₆ est un groupe R₂₀ hydrocarboné, il peut être long et avoir de 12 à 22 atomes de carbone, ou court et avoir de 1 à 3 atomes de carbone.

Lorsque R₁₈ est un groupe R₂₂ hydrocarboné, il a de préférence 1 à 3 atomes de carbone.

Avantageusement, R₁₇, R₁₉ et R₂₁, identiques ou différents, sont choisis parmi les groupes hydrocarbonés en C₁₁-C₂₁, linéaires ou ramifiés, saturés ou insaturés, et plus particulièrement parmi les groupes alkyle et alcényle en C₁₁-C₂₁, linéaires ou ramifiés, saturés ou insaturés.

De préférence, x et z, identiques ou différents, valent 0 ou 1.

Avantageusement, y est égal à 1. De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un groupe méthyle ou éthyle,
- x et y sont égaux à 1 ;
- z est égal à 0 ou 1 ;
- r, s et t sont égaux à 2 ;
- R₁₆ est choisi parmi :
   - le radical 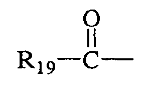
   - les groupes méthyle, éthyle ou hydrocarbonés en C₁₄-C₂₂,
   - l'atome d'hydrogène ;
- R₁₈ est choisi parmi :
   - le radical 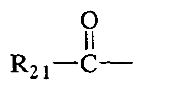
   - l'atome d'hydrogène ;
- R₁₇, R₁₉ et R₂₁, identiques ou différents, sont choisis parmi les groupes hydrocarbonés en C₁₃-C₁₇, linéaires ou ramifiés, saturés ou insaturés, et de préférence parmi les groupes alkyles et alcényles en C₁₃-C₁₇, linéaires ou ramifiés, saturés ou insaturés.

Avantageusement, les groupes hydrocarbonés sont linéaires. On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les groupes acyles ont de préférence 14 à 18 atomes de carbone et proviennent plus particulièrement d'une huile végétale comme l'huile de palme ou de tournesol. Lorsque le composé contient plusieurs groupes acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société COGNIS, STEPANQUAT^{®} par la société STEPAN, NOXAMIUM^{®} par la société CECA, REWOQUAT^{®} WE 18 par la société REWO-WITCO.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les groupes acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (I). On peut notamment citer les chlorures de tétraalkylammonium comme, par exemple, les chlorures de dialkyldiméthylammonium ou d'alkyltriméthylammonium dans lesquels le groupe alkyle comporte environ de 12 à 22 atomes de carbone, en particulier les chlorures de béhényltriméthylammonium, de distéaryldiméthylammonium, de cétyltriméthylammonium, ou de benzyldiméthylstéarylammonium ; le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK, ou encore le chlorure d'hydroxyéthyl-hydroxycétyldimonium (dénomination INCI) commercialisé sous la dénomination DEHYQUART^{®} E-CA par la société COGNIS, le chlorure de dihydroxypropyl-PEG-5-linoléammonium (dénomination INCI) commercialisé sous la dénomination MONAQUAT^{®} SL-5 par la société UNIQEMA et le chlorure-phosphate de linoléamidopropyl PG-dimmonium (dénomination INCI) commercialisé sous la dénomination PHOSPHOLIPID^{®} EFA par la société UNIQEMA.

Le tensioactif cationique particulièrement utilisé dans l'agent microbicide de l'invention est de préférence un sel d'ammonium quaternaire de l'imidazoline répondant à la formule (II) tel que le quaternium-27.

L'agent microbicide selon l'invention contient, de préférence, au moins un tensioactif cationique en une quantité de 20 à 80 % en poids, de préférence de 30 à 60 % en poids par rapport au poids total de l'agent microbicide, ou encore les agents tensioactifs sont contenus en une quantité de 0,05 à 10 % en poids, de préférence de 0,1 à 5 % en poids par rapport au poids total d'une composition contenant l'agent microbicide.

Un agent microbicide particulièrement préféré de la présente invention comprend l'acide anisique, un pentanediol et un quaternium-27 dans les proportions telles que décrites ci-dessus.

L'agent microbicide selon l'invention a notamment une action spécifique contre les bactéries et les champignons tels que les *Aspergillus Niger* et empêche ainsi leur prolifération.

Un autre objet de l'invention consiste en l'utilisation de l'agent microbicide tel que décrit ci-dessus en cosmétique, notamment pour décontaminer une composition cosmétique telle qu'un shampoing.

Cet agent microbicide peut être alors utilisé en une quantité comprise entre 0,1 et 20 % en poids, de préférence entre 1 et 10 % en poids par rapport au poids total de la composition cosmétique.

La composition de traitement cosmétique des matières kératiniques selon l'invention comprend dans un milieu cosmétiquement acceptable, au moins un agent microbicide tel que décrit ci-dessus, en une quantité comprise entre 0,1 et 20 % en poids, de préférence entre 1 et 10 % en poids par rapport au poids total de la composition de traitement cosmétique.

Par milieu cosmétiquement acceptable, on entend un milieu compatible avec toutes les matières kératiniques telles que la peau, les ongles, les cheveux, les cils et sourcils, les lèvres, et toute autre zone cutanée du corps et du visage.

Le milieu aqueux cosmétiquement acceptable peut être constitué par de l'eau ou par un mélange d'eau et d'un ou plusieurs solvants cosmétiquement acceptables tels que les alcools inférieurs en C₁-C₄, par exemple l'éthanol, l'isopropanol, le tertio-butanol, le n-butanol ; les alkylèneglycols comme le propylèneglycol ; les éthers de polyols ; les alcanes en C₅-C₁₀ ; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; ou un de leurs mélanges.

La composition peut également contenir un ou plusieurs des tensioactifs anioniques, non-ioniques ou amphotères bien connus dans la technique.

Comme tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéridesulfates ; les alkylsulfonates, les alkylamidesulfonates, les alkylarylsulfonates, les α-oléfine-sulfonates, les paraffine-sulfonates ; les alkylsulfosuccinates, les alkyléthersulfosuccinates, les alkylamidesulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycosidecarboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éthercarboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les tensioactifs non-ioniques convenant dans l'invention sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL^{®}, tels que décrits dans les brevets US-2 528 378 et US-2 781 354 et classés dans le dictionnaire CTFA, 3^{ème} édition, 1982, sous les dénominations Amphocarboxyglycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₂H,
Rₐ représente un groupe alkyle d'un acide Rₐ-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL^{®} C2M concentré par la société RHODIA.

Les compositions selon l'invention peuvent également contenir un ou plusieurs des additifs classiques bien connus dans la technique, tels que des polymères cationiques, anioniques, non ioniques ou amphotères, des silicones, des paraffines, des esters, des stabilisants polymériques, des épaississants polymériques naturels ou synthétiques, anioniques, amphotères, zwittérioniques, non-ioniques ou cationiques, associatifs ou non, des épaississants non polymériques comme des acides ou des électrolytes, des opacifiants, des parfums, des huiles végétales, minérales et/ou synthétiques, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions cosmétiques selon l'invention peuvent se présenter sous forme de liquides fluides ou épaissis, de gels, de crèmes, de mousses, d'émulsions simples ou d'émulsions multiples.

Elles peuvent être utilisées, par exemple, comme shampoings, soins rincés, masques de soin profond, lotions ou crèmes de traitement du cuir chevelu.

Selon un mode de réalisation préféré de l'invention, la composition peut être utilisée comme shampoing.

La présente invention concerne également un procédé de traitement cosmétique des matières kératiniques, notamment des cheveux qui consiste à appliquer une quantité efficace d'une composition de traitement cosmétique telle que décrite ci-dessus, sur les cheveux, à effectuer un éventuel rinçage après un éventuel temps de pose.

Les exemples suivants illustrent la présente invention et ne doivent être considérés en aucune manière comme limitant l'invention.

### EXEMPLES

### Exemple 1

Des formulations de shampoing ont été préparées à partir des ingrédients indiqués dans le tableau 1 ci-dessous, les proportions étant indiquées en % en poids :

**Tableau 1**

| Composition | | 1 | A | B | C |
|---|---|---|---|---|---|
| Glycérine | | 7,00 | 7,00 | 7,00 | 7,00 |
| 1,2-pentanediol | | 0,10 | 0,10 | 0,10 | - |
| Acide anisique | | 0,20 | 0,20 | - | 0,20 |
| Quaternium-27 (75% M.A.)⁽¹⁾ | | 0,60 | - | 0,60 | - |
| Chlorure de guar-hydroxy-propyltrimonium | | - | 0,05 | - | - |
| Distéarate de PEG-150 | | 4,20 | 3,50 | 5,00 | 3,75 |
| Décylglucoside (50% M.A.) | | 30,00 | 30,00 | 30,00 | 30,00 |
| Chlorure de sodium | | 3,00 | 3,00 | 3,00 | 3,00 |
| Acide citrique | | 2,40 | 2,40 | 2,40 | 2,40 |
| Hydroxyde de sodium | | 0,92 | 0,94 | 0,95 | 1,05 |
| Eau | qsp | 100 | 100 | 100 | 100 |
| | pH | 5,2 | 5,2 | 5,2 | 5,2 |

| | | | | | |
|---|---|---|---|---|---|
| (1) commercialisé par REWO sous le nom REWOQUAT^{®} W75 | | | | | |

La composition 1 est une composition selon l'invention. Les compositions A, B et C ne contiennent que certains constituants de l'agent microbicide.

Pour déterminer l'efficacité bactéricide de l'agent microbicide selon la présente invention dans un shampoing, on a utilisé le test tel que décrit dans European Pharmacopoeia - supplément 2001, pages 293-294. Ce test a permis d'observer l'évolution du nombre de micro-organismes, ici les champignons *Aspergillus Niger,* au cours du temps dans les quatre compositions.

On a contaminé chacune des compositions 1, A, B et C avec une quantité de champignons *Aspergillus Niger* en suspension dans une solution contenant 9 g/l de chlorure de sodium et 0,5 g/l de polysorbate 80, le volume de la suspension ne dépassant pas 1 % du volume de chaque composition.

Après avoir mélangé énergiquement les compositions inoculées pour obtenir une répartition uniforme des micro-organismes dans celles-ci, on a prélevé 1 ml de chaque composition inoculée et déterminé le nombre de micro-organismes, cette première mesure correspondant à t = 0 jour, puis on les a conservées à l'abri de la lumière à une température de 20 à 25 °C.

On a ensuite déterminé le nombre de micro-organismes au bout de 7 jours et de 14 jours. Les résultats sont indiqués dans le tableau 2 ci-dessous.

**Tableau 2**

| *Aspergillus Niger* (nombre de micro-organismes/ml de composition) | | | | |
|---|---|---|---|---|
| 0 jour | 5,1x10⁵ | 2,8x10⁵ | 5,1x10⁵ | 1,4x10⁵ |
| 7 jours | 3,0x10² | 9,0x10⁴ | 4,3x10⁵ | 5,9x10⁴ |
| 14 jours | < 100 | 1,2x10⁴ | 2,9x10⁵ | 6,5x10³ |

On observe une réduction très significative du nombre de champignons *Aspergillus Niger* dans la composition selon l'invention, par rapport aux compositions A, B et C.

### Exemples 2 à 4

D'autres compositions selon l'invention ont été préparées à partir des ingrédients présentés dans le tableau 3 ci-dessous, les proportions étant indiquées en % en poids.

**Tableau 3**

| Exemple | | 2 | 3 | 4 |
|---|---|---|---|---|
| Acide anisique | | 0,2 | 0,2 | 0,2 |
| 1,2-pentanediol | | 0,1 | 0,1 | 0,1 |
| Quaternium-27 (75% M.A.)⁽¹⁾ | | 0,45 (M.A.) | 0,45 (M.A.) | 0,45 (M.A.) |
| Distéarate de PEG-150 | | 4,4 | 4 | 3,5 |
| PEG-4 | | 7 | | |
| PEG-8 | | | 7 | |
| Sorbitol (70 % M.A.) | | | | 7 (M.A.) |
| Décylglucoside (50% M.A.) | | 15 (M.A.) | 15 (M.A.) | 15 (M.A.) |
| Chlorure de sodium | | 3 | 3 | 3 |
| Acide citrique | | 2,4 | 2,4 | 2,4 |
| Eau | qsp | 100 | 100 | 100 |
| Hydroxyde de sodium | qs pH | 5,2 | 5,2 | 5,2 |

| | | | | |
|---|---|---|---|---|
| ⁽¹⁾ commercialisé par REWO sous le nom REWOQUAT® W75 | | | | |

## Revendications

1. Agent microbicide comprenant l'acide anisique, un de ses sels ou un de ses esters d'alkyle en C₁₋₄, au moins un polyol présentant une chaîne hydrocarbonée qui comporte au moins 4 atomes de carbone et qui porte au moins deux groupes hydroxy, et au moins un tensioactif cationique.

2. Agent microbicide selon la revendication 1, **caractérisé en ce que** le sel d'acide anisique est choisi parmi un sel de métal alcalin ou alcalino-terreux, un sel d'ammonium ou un sel avec une amine organique.

3. Agent microbicide selon la revendication 1 ou 2, **caractérisé en ce que** le polyol présente une chaîne hydrocarbonée comportant de 4 à 50 atomes de carbone, et portant de 2 à 10 groupes hydroxy, et présente une masse moléculaire moyenne en poids inférieure ou égale à 1000.

4. Agent microbicide selon la revendication 3, **caractérisé en ce que** le polyol est choisi parmi les polyalkylèneglycols, les pentanediols, le sorbitol ou leurs mélanges.

5. Agent microbicide selon l'une quelconque des revendications précédentes, **caractérisé en ce que** le tensioactif cationique est choisi parmi :
- ceux qui présentent la formule générale (I) suivante : 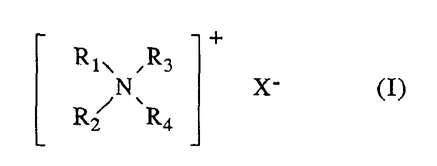 dans laquelle les symboles R₁ à R₄, qui peuvent être identiques ou différents, représentent un groupe aliphatique, linéaire ou ramifié, comportant de 1 à 30 atomes de carbone, ou un groupe aromatique tel que aryle ou alkylaryle ; X⁻ est un anion choisi dans l'ensemble constitué par les halogénures, les phosphates, les acétates, les lactates, alkyl(C₂-C₆)sulfates, les alkyl- ou alkylaryl-sulfonates ;
- les sels d'ammonium quaternaire de l'imidazoline ;
- les sels de diammonium quaternaire de formule (III) : 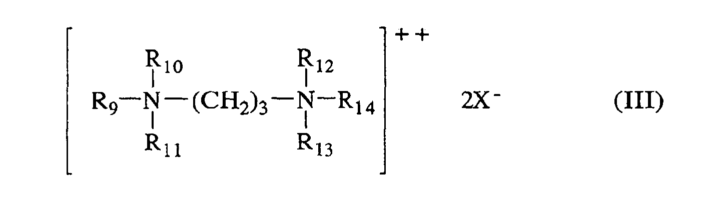 dans laquelle R₉ désigne un groupe aliphatique comportant environ de 16 à 30 atomes de carbone, R₁₀, R₁₁, R₁₂, R₁₃ et R₁₄, identiques ou différents représentent chacun un atome d'hydrogène ou un groupe alkyle comportant de 1 à 4 atomes de carbone, et X⁻ est un anion choisi dans l'ensemble constitué par les halogénures, les acétates, les phosphates, les nitrates et les méthylsulfates ; et
- les sels d'ammonium quaternaire contenant au moins une fonction ester.

6. Agent microbicide selon la revendication 5, **caractérisé en ce que** le tensioactif cationique est le quaternium-27.

7. Agent microbicide selon l'une quelconque des revendications précédentes, **caractérisé en ce que** l'acide anisique, un de ses sels ou un de ses esters est contenu en une quantité de 1 à 30 % en poids, de préférence de 4 à 20 % en poids par rapport au total de l'agent microbicide.

8. Agent microbicide selon l'une quelconque des revendications précédentes, **caractérisé en ce que** l'acide anisique, un de ses sels ou un de ses esters est contenu en une quantité allant jusqu'à 1 % en poids, de préférence en une quantité de 0,1 à 0,4 % en poids par rapport au poids total d'une composition contenant l'agent microbicide.

9. Agent microbicide selon l'une quelconque des revendications précédentes, **caractérisé en ce que** le polyol est contenu en une quantité allant jusqu'à 50 % en poids, de préférence en une quantité de 10 à 30 % en poids par rapport au poids total de l'agent microbicide.

10. Agent microbicide selon l'une quelconque des revendications précédentes, **caractérisé en ce que** le polyol est contenu en une quantité allant jusqu'à 10 % en poids, de préférence en une quantité de 5 à 10 % en poids par rapport au poids total d'une composition contenant l'agent microbicide.

11. Agent microbicide selon l'une quelconque des revendications précédentes, **caractérisé en ce que** le tensioactif cationique est contenu en une quantité de 20 à 80 % en poids, de préférence de 30 à 60 % en poids par rapport au poids total de l'agent microbicide.

12. Agent microbicide selon l'une quelconque des revendications précédentes, **caractérisé en ce que** le tensioactif cationique est contenu en une quantité de 0,05 à 10 % en poids, de préférence de 0,1 à 5 % en poids par rapport au poids total d'une composition contenant l'agent microbicide.

13. Agent microbicide selon l'une quelconque des revendications précédentes, **caractérisé en ce qu'**il comprend l'acide anisique, un pentane-diol et le quaternium-27

14. Utilisation d'un agent microbicide selon l'une quelconque des revendications précédentes, pour empêcher la prolifération des bactéries et des champignons dans des compositions cosmétiques.

15. Utilisation selon la revendication 14 pour décontaminer une composition cosmétique.

16. Composition de traitement cosmétique des matières kératiniques, comprenant dans un milieu cosmétiquement acceptable, au moins un agent microbicide selon l'une quelconque des revendications précédentes.

17. Composition selon la revendication 16, **caractérisée en ce que** l'agent microbicide est contenu en une quantité comprise entre 0,1 et 20 % en poids par rapport au poids total de la composition.

18. Composition selon la revendication 16 ou 17, **caractérisée en ce que** le milieu cosmétiquement acceptable est constitué par de l'eau ou par un mélange d'eau et d'un ou plusieurs solvants cosmétiquement acceptables.

19. Composition selon la revendication 18, **caractérisée en ce que** les solvants cosmétiquement acceptables sont choisis parmi les alcools inférieurs en C₁-C₄, les alkylèneglycols, les éthers de polyols, les alcanes en C₅-C₁₀, l'acétone, la méthyléthylcétone, les acétates d'alkyle en C₁-C₄, le diméthoxyéthane, le diéthoxyéthane, ou un de leurs mélanges.

20. Composition selon l'une quelconque des revendications 16 à 19, **caractérisée en ce qu'**elle comprend un ou plusieurs des tensioactifs anioniques, non-ioniques ou amphotères.

21. Composition selon l'une quelconque des revendications 16 à 20, **caractérisée en ce qu'**elle comprend un ou plusieurs des additifs tels que des polymères cationiques, anioniques, non ioniques ou amphotères, des silicones, des paraffines, des esters, des stabilisants polymériques, des épaississants polymériques naturels ou synthétiques, anioniques, amphotères, zwittérioniques, non-ioniques ou cationiques, associatifs ou non, des épaississants non polymériques comme des acides ou des électrolytes, des opacifiants, des parfums, des huiles végétales, minérales et/ou synthétiques, des colorants, des particules organiques ou minérales, des conservateurs, des agents de stabilisation du pH.

22. Composition selon l'une quelconque des revendications 16 à 21, **caractérisée ce qu'**elle est utilisée comme shampoing, soin rincé, masque de soin profond, lotion ou crème de traitement du cuir chevelu.

23. Procédé de traitement cosmétique, **caractérisé en ce qu'**il consiste à appliquer une quantité efficace d'une composition de traitement cosmétique selon l'une quelconque des revendications 16 à 22, sur les cheveux, à effectuer un éventuel rinçage après un éventuel temps de pose.

## Claims

1. Microbicidal agent comprising anisic acid, one of its salts or one of its C₁₋₄ alkyl esters, at least one polyol having a hydrocarbon chain which comprises at least 4 carbon atoms and which carries at least two hydroxyl groups and at least one cationic surfactant.

2. Microbicidal agent according to Claim 1, **characterized in that** the anisic acid salt is chosen from an alkali or alkaline-earth metal salt, an ammonium salt or a salt with an organic amine.

3. Microbicidal agent according to Claim 1 or 2, **characterized in that** the polyol has a hydrocarbon chain comprising from 4 to 50 carbon atoms and carrying from 2 to 10 hydroxyl groups, and has a weight-average molecular mass of less than or equal to 1000.

4. Microbicidal agent according to Claim 3,
**characterized in that** the polyol is chosen from polyalkylene glycols, pentanediols, sorbitol or mixtures thereof.

5. Microbicidal agent according to any one of the preceding claims, **characterized in that** the cationic surfactant is chosen from:
- those which have the following general formula (I): 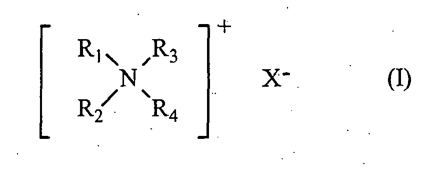 in which the symbols R₁ to R₄, which may be identical or different, represent a linear or branched aliphatic group comprising from 1 to 30 carbon atoms, or an aromatic group such as aryl or alkylaryl; X⁻ is an anion chosen from the group consisting of halides, phosphates, acetates, lactates, (C₂-C₆)alkyl sulphates, alkyl or alkylaryl sulphonates;
- the quaternary ammonium salts of imidazoline;
- the quaternary diammonium salts of formula (III): 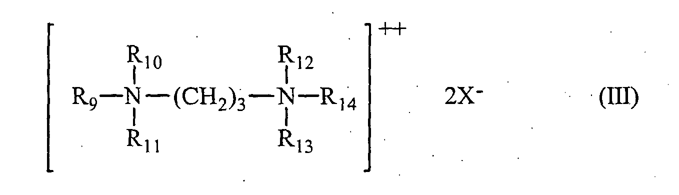 in which R₉ denotes an aliphatic group comprising about from 16 to 30 carbon atoms, R₁₀, R₁₁, R₁₂, R₁₃ and R₁₄, which are identical or different, each represent a hydrogen atom or an alkyl group comprising from 1 to 4 carbon atoms, and X- is an anion chosen from the group consisting of halides, acetates, phosphates, nitrates and methyl sulphates; and
- the quaternary ammonium salts containing at least one ester functional group.

6. Microbicidal agent according to Claim 5, **characterized in that** the cationic surfactant is quaternium-27.

7. Microbicidal agent according to any one of the preceding claims, **characterized in that** the anisic acid, one of its salts or one of its esters is contained in a quantity from 1 to 30% by weight, preferably from 4 to 20% by weight relative to the total weight of the microbicidal agent.

8. Microbicidal agent according to any one of the preceding claims, **characterized in that** the anisic acid, one of its salts or one of its esters is contained in a quantity ranging up to 1% by weight, preferably in a quantity from 0.1 to 0.4% by weight relative to the total weight of a composition containing the microbicidal agent.

9. Microbicidal agent according to any one of the preceding claims, **characterized in that** the polyol is contained in a quantity ranging up to 50% by weight, preferably in a quantity from 10 to 30% by weight relative to the total weight of the microbicidal agent.

10. Microbicidal agent according to any one of the preceding claims, **characterized in that** the polyol is contained in a quantity ranging up to 10% by weight, preferably in a quantity from 5 to 10% by weight relative to the total weight of a composition containing the microbicidal agent.

11. Microbicidal agent according to any one of the preceding claims, **characterized in that** the cationic surfactant is contained in a quantity from 20 to 80% by weight, preferably from 30 to 60% by weight relative to the total weight of the microbicidal agent.

12. Microbicidal agent according to any one of the preceding claims, **characterized in that** the cationic surfactant is contained in a quantity from 0.05 to 10% by weight, preferably from 0.1 to 5% by weight relative to the total weight of a composition containing the microbicidal agent.

13. Microbicidal agent according to any one of the preceding claims, **characterized in that** it comprises anisic acid, a pentanediol and quaternium-27.

14. Use of a microbicidal agent according to any one of the preceding claims, for preventing the proliferation of bacteria and fungi in cosmetic compositions.

15. Use according to Claim 14 for decontaminating a cosmetic composition.

16. Composition for the cosmetic treatment of keratinous materials, comprising, in a cosmetically acceptable medium, at least one microbicidal agent according to any one of the preceding claims.

17. Composition according to Claim 16, **characterized in that** the microbicidal agent is contained in a quantity of between 0.1 and 20% by weight relative to the total weight of the composition.

18. Composition according to Claim 16 or 17, **characterized in that** the cosmetically acceptable medium consists of water or of a mixture of water and of one or more cosmetically acceptable solvents.

19. Composition according to Claim 18, **characterized in that** the cosmetically acceptable solvents are chosen from C₁-C₄ lower alcohols, alkylene glycols, polyol ethers, C₅-C₁₀ alkanes, acetone, methyl ethyl ketone, C₁-C₄ alkyl acetates, dimethoxyethane, diethoxyethane, or one of the mixtures thereof.

20. Composition according to any one of Claims 16 to 19, **characterized in that** it comprises one or more anionic, nonionic or amphoteric surfactants.

21. Composition according to any one of Claims 16 to 20, **characterized in that** it comprises one or more additives such as cationic, anionic, nonionic or amphoteric polymers, silicones, paraffins, esters, polymeric stabilizers, natural or synthetic polymeric thickeners, which are anionic, amphoteric, zwitterionic, nonionic or cationic, associative or non-associative, nonpolymeric thickeners such as acids or electrolytes, opacifiers, perfumes, vegetable, mineral and/or synthetic oils, colorants, organic or inorganic particles, preservatives, pH-stabilizing agents.

22. Composition according to any one of Claims 16 to 21, **characterized in that** it is used as a shampoo, rinse-off treatment, deep treatment mask, lotion or cream for treating the scalp.

23. Method of cosmetic treatment, **characterized in that** it consists in applying to the hair an effective quantity of a composition for cosmetic treatment according to any one of Claims 16 to 22, and in optionally rinsing after a possible exposure time.

## Patentansprüche

1. Mikrobiozides Mittel, das Anissäure, eines ihrer Salze oder einen ihrer C₁₋₄-Alkylester, mindestens ein Polyol, das eine Kohlenwasserstoffkette aufweist, die mindestens 4 Kohlenstoffatome besitzt und mindestens zwei Hydroxygruppen trägt, und mindestens einen kationischen grenzflächenaktiven Stoff enthält.

2. Mikrobiozides Mittel nach Anspruch 1, **dadurch gekennzeichnet, dass** das Salz der Anissäure unter den Alkalimetallsalzen, Erdalkalimetallsalzen, Ammoniumsalzen oder den Salzen eines organischen Amins ausgewählt ist.

3. Mikrobiozides Mittel nach Anspruch 1 oder 2, **dadurch gekennzeichnet, dass** das Polyol eine Kohlenwasserstoffkette aufweist, die 4 bis 50 Kohlenstoffatomen aufweist und 2 bis 10 Hydroxygruppen trägt, und eine gewichtsmittlere Molmasse von höchstens 1000 besitzt.

4. Mikrobiozides Mittel nach Anspruch 3, **dadurch gekennzeichnet, dass** das Polyol unter den Polyalkylenglycolen, Pentandiolen, Sorbit oder deren Gemischen ausgewählt ist.

5. Mikrobiozides Mittel nach einem der vorhergehenden Ansprüche, **dadurch gekennzeichnet, dass** der kationische grenzflächenaktive Stoff ausgewählt ist unter:
- Salzen, die die folgende allgemeine Formel (I) aufweisen: 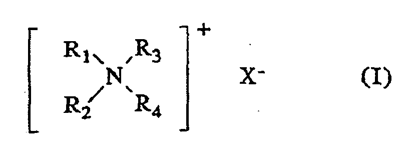 worin die Gruppen R₁ bis R₄, die gleich oder verschieden sein können, geradkettige oder verzweigte aliphatische Gruppen mit 1 bis 30 Kohlenstoffatomen oder aromatische Gruppen, wie Aryl oder Alkylaryl bedeuten; X- ein Anion ist, das unter den Halogeniden, Phosphaten, Acetaten, Lactaten, Alkyl(C₂₋₆)sulfaten, Alkyl- oder Alkylarylsulfonaten ausgewählt ist;
- quartären Ammoniumsalzen von Imidazolin;
- quartären Diammoniumsalzen der Formel (III): 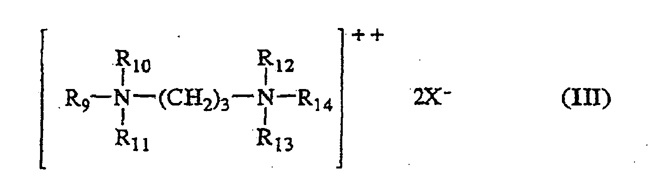 worin die Gruppe R₉ eine aliphatische Gruppe mit etwa 16 bis 30 Kohlenstoffatomen bedeutet, die Gruppen R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind, und X- ein Anion ist, das unter den Halogeniden, Acetaten, Phosphaten, Nitraten und Methylsulfaten ausgewählt ist;
- quartären Ammoniumsalzen, die mindestens eine Esterfunktion enthalten.

6. Mikrobiozides Mittel nach Anspruch 5, **dadurch gekennzeichnet, dass** der grenzflächenaktive Stoff das Quaternium-27 ist.

7. Mikrobiozides Mittel nach einem der vorhergehenden Ansprüche, **dadurch gekennzeichnet, dass** die Anissäure, eines ihrer Salze oder ein Ester von Anissäure in einer Menge von 1 bis 30 Gew.-% und insbesondere 4 bis 20 Gew.-%, bezogen auf das Gesamtgewicht des mikrobioziden Mittels, enthalten ist.

8. Mikrobiozides Mittel nach einem der vorhergehenden Ansprüche, **dadurch gekennzeichnet, dass** die Anissäure, das Salz von Anissäure oder der Ester von Anissäure in einer Menge von bis zu 1 Gew.-% und vorzugsweise 0,1 bis 0,4 Gew.-%, bezogen auf das Gesamtgewicht einer Zusammensetzung, die das mikrobiozide Mittel enthält, enthalten ist.

9. Mikrobiozides Mittel nach einem der vorhergehenden Ansprüche, **dadurch gekennzeichnet, dass** das Polyol in einer Menge von bis zu 50 Gew.-% und vorzugsweise 10 bis 30 Gew.-%, bezogen auf das Gesamtgewicht des mikrobioziden Mittels, enthalten ist.

10. Mikrobiozides Mittel nach einem der vorhergehenden Ansprüche, **dadurch gekennzeichnet, dass** das Polyol in einer Menge von bis zu 10 Gew.-% und vorzugsweise 5 bis 10 Gew.-%, bezogen auf das Gesamtgewicht einer Zusammensetzung, die das mikrobiozide Mittel enthält, vorliegt.

11. Mikrobiozides Mittel nach einem der vorhergehenden Ansprüche, **dadurch gekennzeichnet, dass** der kationische grenzflächenaktive Stoff in einer Menge von 20 bis 80 Gew.-% und vorzugsweise 30 bis 60 Gew.-%, bezogen auf das Gesamtgewicht des mikrobioziden Mittels, enthalten ist.

12. Mikrobiozides Mittel nach einem der vorhergehenden Ansprüche, **dadurch gekennzeichnet, dass** der kationische grenzflächenaktive Stoff in einer Menge von 0,05 bis 10 Gew.-% und vorzugsweise 0,1 bis 5 Gew.-%, bezogen auf das Gesamtgewicht einer Zusammensetzung, die das mikrobiozide Mittel enthält, vorliegt.

13. Mikrobiozides Mittel nach einem der vorhergehenden Ansprüche, **dadurch gekennzeichnet, dass** es Anissäure, ein Pentandiol und Quaternium-27 enthält.

14. Verwendung eines mikrobioziden Mittels nach einem der vorhergehenden Ansprüche, um die starke Vermehrung von Bakterien und Pilzen in kosmetische Zusammensetzungen zu verhindern.

15. Verwendung nach Anspruch 14, um eine kosmetische Zusammensetzung zu dekontaminieren.

16. Zusammensetzung für die kosmetische Behandlung von Keratinsubstanzen, die in einem kosmetisch akzeptablen Medium mindestens ein mikrobiozides Mittel nach einem der vorhergehenden Ansprüche enthält.

17. Zusammensetzung nach Anspruch 16, **dadurch gekennzeichnet, dass** das mikrobiozide Mittel in einer Menge von 0,1 bis 20 Gew.-%, bezogen auf das Gesamtgewicht der Zusammensetzung, enthalten ist.

18. Zusammensetzung nach Anspruch 16 oder 17, **dadurch gekennzeichnet, dass** das kosmetisch akzeptable Medium aus Wasser oder einem Gemisch von Wasser und einem oder mehreren kosmetisch akzeptablen Lösungsmitteln besteht.

19. Zusammensetzung nach Anspruch 18, **dadurch gekennzeichnet, dass** die kosmetisch akzeptablen Lösungsmittel unter niederen C₁₋₄-Alkoholen, Alkylenglycolen, Polyolethern, C₅₋₁₀-Alcanen, Aceton, Methylethylketon, C₁-₄-Alkylacetaten, Dimethoxyethan, Diethoxyethan und deren Gemischen ausgewählt sind.

20. Zusammensetzung nach einem der Ansprüche 16 bis 19, **dadurch gekennzeichnet, dass** sie einen oder mehrere anionische, nichtionische oder amphotere grenzflächenaktive Stoffe enthält.

21. Zusammensetzung nach einem der Ansprüche 16 bis 20, **dadurch gekennzeichnet, dass** sie einen oder mehrere Zusatzstoffe enthält, wie kationische, anionische, nichtionische oder amphotere Polymere, Silicone, Paraffine, Ester, polymere Stabilisatoren, natürliche oder synthetische, anionische, amphotere, zwitterionische, nichtionische oder kationische, assoziative oder nicht assoziative, polymere Verdickungsmittel, nichtpolymere Verdickungsmittel, wie beispielsweise Säuren oder Elektrolyte, Trübungsmittel, Parfums, pflanzliche Öle, Mineralöle und/oder synthetische Öle, Farbmittel, organische oder anorganische Partikel, Konservierungsmittel, pH-Stabilisatoren.

22. Zusammensetzung nach einem der Ansprüche 16 bis 21, **dadurch gekennzeichnet, dass** sie als Haarwaschmittel, Pflegeprodukt, das ausgespült wird, Maske für die Tiefenpflege, Lotion oder Creme zur Behandlung der Kopfhaut verwendet wird.

23. Verfahren zur kosmetischen Behandlung, **dadurch gekennzeichnet, dass** es darin besteht, eine wirksame Menge einer Zusammensetzung zur kosmetischen Behandlung nach einem der Ansprüche 16 bis 22 auf die Haare aufzutragen und nach einer gegebenenfalls abgewarteten Einwirkzeit gegebenenfalls zu spülen.
